(19) Europäisches Patentamt
European Patent Office
Office européen des brevets

(11) Veröffentlichungsnummer: **0 257 320**
**A2**

# (12) EUROPÄISCHE PATENTANMELDUNG

(21) Anmeldenummer: **87110843.7**

(22) Anmeldetag: **27.07.87**

(51) Int. Cl.[4]: **A61K 37/12**, A61K 47/00

(30) Priorität: **08.08.86 DE 3626868**

(43) Veröffentlichungstag der Anmeldung:
**02.03.88 Patentblatt 88/09**

(84) Benannte Vertragsstaaten:
**AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Serno, Peter, Dr.**
**An der Ruthen 3**
**D-5000 Köln 80(DE)**
Erfinder: **Detzer, Klaus, Dr.**
**Überdorf 6**
**D-5307 Wachtberg(DE)**
Erfinder: **Winter, Manfred, Dr.**
**Roggendorfstrasse 49**
**D-5000 Köln 80(DE)**

## (54) Parenterale Lösung.

(57) Parenterale Lösungen von schwer löslichen Arzneimitteln enthalten modifizierte Gelatine.

EP 0 257 320 A2

## Parenterale Lösung

Die parenterale Applikation von Arzneistoffen, im wesentlichen die intravenöse Applikation, ist nur in gelöster Form möglich. Daher bereitet die Formulierung von Injektions-und Infusionslösungen bei Arzneistoffen geringer Wasserlöslichkeit regelmäßig Schwierigkeiten.

Im Falle unzureichender Löslichkeit in Wasser wurden bislang bei der Formulierung parenteraler Lösungen schwer löslicher Anzneistoffe organische Lösungsmittel wie Propylenglykol, Polyethylenglykol, Ethanol, Glycerin-Polyethylenglykolricinoleat (Cremophor® EL) oder Polyoxyethylensorbitanfettsäureester (Tween®) zur Erhöhung der Löslichkeit zugesetzt. Die Effektivität dieser Maßnahme wird allerdings durch begrenzt, daß Lösungsmittel nur in niederen Konzentrationen angewendet werden können, da höhere Konzentration zu unerwünschten Nebenwirkungen wie Injektionsschmerz, Thrombophlebitis und Venenverödung führen. Darüber hinaus führen einige Lösungsmittel zu Nebenwirkungen wie anaphylaktische Schocks und Hämolyse.

Eine andere Möglichkeit der Lösungsvermittlung von Arzneistoffen geringer Wasserlöslichkeit besteht darin, den Arzneistoff in der Fettphase in einer Emulsion zu lösen (US 4 073 943). Dieses Verfahren setzt allerdings eine sehr gute Löslichkeit des Arzneistoffs in physiologisch verträglichen Ölen wie Sojabohnenöl voraus, die nur in den seltensten Fällen gewährleistet ist.

Die in US 4 158 707 beschriebene Lösungsvermittlung mit einer Kombination aus Gallensäure und Lipoid hat den Nachteil einer begrenzten Haltbarkeit der Lösungsvermittler, insbesondere bei höheren Temperaturen, sowie von Nebenwirkungen wie Erbrechen, Hämolyse und Cholestasis bei Gaben in höheren Dosen.

Es wurden parenterale Lösungen von schwer löslichen Arzneimitteln gefunden, die modifizierte Gelatine enthalten.

Parenterale Lösungen sind hierbei im wesentlichen intravenöse Applikationsformen von Arzneistoffen, im besonderen Injektions-und Infusionslösungen. Für die erfindungsgemäßen parenteralen Lösungen verwendet man im allgemeinen modifizierte Gelatine mit einem Molekulargewicht im Bereich von 10.000 bis 50.000 wie Oxypolygelatine oder Polymerisat abgebauter succinoylierter Gelatine.

Die modifizierte Gelatine ist an sich bekannt und kann beispielsweise Oxypolygelatine, Polygeline oder Succinoyl-Gelatine darstellen.

Oxypolygelatine wird durch Decalcifizieren von Gelatine, Kondensation mit Glyoxal und Oxidation mit Wasserstoffperoxid hergestellt und ist von Campbell, D. H. et al., Texas Rep. Biol. Med. 9, 235 (1951), Bonhard, K., Arzneim.-Forsch. 21, 1667 (1971) sowie Nitschmann und Stoll, Pharm.-Ztg. 42, 1594 (1968) beschrieben.

Polygeline wird durch Vernetzen von Rindergelatine mit Hexamethylendiisocyanat gewonnen und wird von Schmidt-Thome, J., A. Mager und H. H. Schöne, Arzneim.-Forsch. 12, 378 (1962) beschrieben.

Succinoyl-Gelatine wird durch Kondensation von Gelatine und Bernsteinsäureanhydrid gewonnen und ist in US 2 827 419, GB 836 082 sowie von Tourtelotte, D. und H. E. Williams, in: Stainsby, G., Gelatin and Glue Research, 1958 beschrieben.

Bevorzugt bezieht sich die vorliegende Erfindung auf eine neue Form der Injektion schwer löslicher Arzneistoffe bestehend aus einer Konzentrat-und einer Verdünnungslösung, die vor Applikation gemischt werden und dabei hinreichend stabile, übersättigte Lösungen bilden.

Schwer lösliche Arzneistoffe für die vorliegende Erfindung haben im allgemeinen eine Löslichkeit in Wasser zwischen 1 µg und 10 g, bevorzugt 10 µg and 1 g, pro Liter.

Schwer lösliche Arzneistoffe für die erfindungsgemäßen parenteralen Lösungen sind beispielsweise
Benzodiazepine wie Diazepam, Flunitrazepam,
Antiepilektika wie Diphenylhydantoin, Clonazepam,
Chemotherapeutika wie Nitrofurantoin, Sulfamethoxazol, Trimethoprim,
Antimykotika wie Griseofulvin, Amphotericin B, Ketoconazol,
Herzglykoside wie Digoxin, Deslanosid,
Mutterkornalkaloide wie Dihydroergotaminmesilat, Ergotamintartrat,
Zytostatika wie Melphalan,
Barbiturate wie Pentobarbital-Natrium
sowie fettlösliche Vitamine wie Vitamin A, $B_2$, $B_6$, $B_{12}$, E oder $K_1$.

Von ganz besonderer Bedeutung sind die Dihydropyridinverbindungen, insbesondere die mit folgender allgemeiner Formel

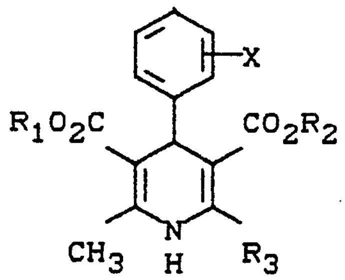

(I)

in der

$R_1$ $C_1$-$C_4$-Alkyl, gegebenenfalls substituiert durch $C_1$-$C_3$-Alkoxy,
$R_2$ $C_1$-$C_{10}$-Alkyl, gegebenenfalls substituiert durch $C_1$-$C_3$-Alkoxy, Trifluormethyl, N-Methyl-N-benzyl-amino,
$R_3$ $C_1$-$C_4$-Alkyl, Cyano, Hydroxymethyl und
X 2-bzw. 3-Nitro, 2,3-Dichlor, 2,3 Ringglied bestehend aus =N-O-N=,
bedeuten.

Ganz besonders in Betracht kommen die Verbindungen der folgenden Tabelle:

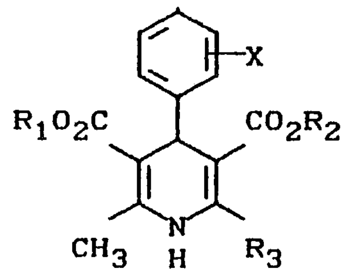

(I)

| Nr. | X | $R^1$ | $R^2$ | $R^3$ | Generic |
|---|---|---|---|---|---|
| 1 | 2-$NO_2$ | $CH_3$ | $CH_3$ | $CH_3$ | Nifedipin |
| 2 | 3-$NO_2$ | $nPrOCH_2CH_2$ | $nPrOCH_2CH_2$ | $CH_3$ | Niludipin |
| 3 | 3-$NO_2$ | $C_2H_5$ | $CH_3$ | $CH_3$ | Nitrendipin |
| 4 | 2-$NO_2$ | $CH_3$ | $(CH_3)_2CHCH_2$ | $CH_3$ | Nisoldipin |
| 5 | 3-$NO_2$ | $CH(CH_3)_2$ | $(CH_2)_2$-O-$CH_3$ | $CH_3$ | Nimodipin |
| 6 | 3-$NO_2$ | $C_2H_5$ | $C_{10}H_{21}$(n) | $CH_3$ | |
| 7 | 2-Cl | $CH_3$ | $CH_2$-$CF_3$ | $CH_3$ | |
| 8 | 2-Cl | $C_2H_5$ | $CH_2$-$CF_3$ | $CH_3$ | |
| 9 | 3-$NO_2$ | $CH(CH_3)_2$ | n-PrO-$CH_2CH_2$ | $CH_3$ | |
| 10 | 3-$NO_2$ | $CH_3$ | $C_6H_5CH_2N(CH_3)CH_2CH_2$ | $CH_3$ | Nicardipin |
| 11 | 2,3-$Cl_2$ | $C_2H_5$ | $CH_3$ | $CH_3$ | Felodipin |
| 12 | 2,3=N-O-N= | $C_2H_5$ | $C_2H_5$ | $CH_3$ | |
| 13 | 2,3=N-O-N= | $CH_3$ | $CH(CH_3)_2$ | $CH_3$ | |
| 14 | 3-$NO_2$ | $C_2H_5$ | $C_2H_5$ | $CH_2OH$ | |
| 15 | 3-$NO_2$ | $CH_3$ | $CH_3$ | CN | |
| 16 | 3-$NO_2$ | $CH_3$ | $(CH_2)_3$-$(CF_2)_5$-$CF_3$ | $CH_3$ | |

n-Pr = n-Propyl

Ebenfalls von Bedeutung ist der Wirkstoff:

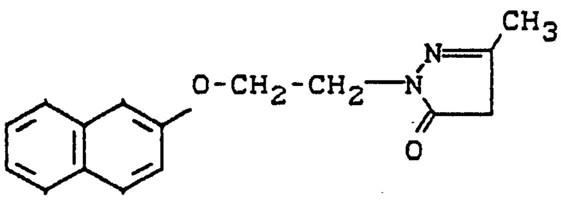

Nafazatrom

Weitere Bestandteile des Konzentrates sind ein oder mehrere zur intravenösen Injektion grundsätzlich geeignete organische Lösungsmittel wie Ethanol, 1,2-Propylenglykol, Polyethylenglykol mit Molekulargewichten zwischen 200 und 600, Glycerin oder Tetrahydrofurfurylalkoholpolyethylenglykolether in Konzentrationen von 30 bis 99,999 % G/V.

Das Konzentrat kann 0,1 bis 30 % G/V modifizierte Gelatine mit einem Molekulargewicht zwischen 10.000 und 50.000 wie Oxypolygelatine oder Polymerisat abgebauter succinoylierter Gelatine und/oder bis zu 70 % G/V Wasser für Injektionszwecke enthalten. Darüber hinaus können weitere, in parenteralen Lösungen übliche Hilfsstoffe zugesetzt werden.

Die Verdünnungslösung enthält 0,1 bis 30 % G/V modifizierte Gelatine mit einem Molekulargewicht zwischen 10.000 und 50.000 wie Oxypolygelatine oder Polymerisat abgebauter succinoylierter Gelatine, 10 bis 99,9 % G/V Wasser für Injektionszwecke soeie gegebenenfalls weitere, in parenteralen Lösungen übliche Hilfsstoffe.

Übliche Hilfsstoffe für parenterale Lösungen sind beispielsweise:
Säuren, Basen oder Puffersubstanzen zur pH-Einstellung, Salze, Zucker oder mehrwertige Alkohole zur Isotonisierung, Konservierungsmittel wie Benzylalkohol, Chlorbutanol, Antioxidantien wie Sulfite, Acetylcystein oder Ascorbinsäure.

Enthält das Konzentrat bereits modifizierte Gelatine, kann dies in der Verdünnungslösung entfallen.

Es wurde auch ein Verfahren zur Herstellung der erfindungsgemäßen parenteralen Lösungen von schwer löslichen Arzneimitteln gefunden, das dadurch gekennzeichnet ist, daß man das Arzneimittel in einem Lösungsmittel löst und dann Wasser, modifizierte Gelatine und übliche Hilfsstoffe zusetzt.

In einer bevorzugten Ausführungsform des erfindungsgemäßen Verfahrens stellt man eine Konzentrat- und eine Verdünnungslösung her, die vor Applikation gemischt werden und dabei eine stabile, übersättigte Lösung bilden.

Die bevorzugte Ausführungsform des erfindungsgemäßen Verfahrens ist dadurch gekennzeichnet, daß man zur Herstellung der Konzentratlösung das Arzneimittel unter Rühren in einem organischen Lösungsmittel löst und dann Wasser, modifizierte Gelatine und übliche Hilfsstoffe zusetzt und zur Herstellung der Verdünnungslösung modifizierte Gelatine in Wasser löst und dann die Konzentratlösung und die Verdünnungslösung mischt.

Organische Lösungsmittel zur Lösung der schwer löslichen Arzneimittel sind beispielsweise:
Ethanol, 1,2-Propylenglykol, Polyethylenglykol mit Molekulargewichten zwischen 200 und 600, Glycerin oder Tetrahydrofurfurylalkoholpolyethylenglykolether.

Im allgemeinen mischt man die Konzentrat-und die Verdünnungslösung im Verhältnis 1 Volumenteil der Konzentratlösung zu 0,2 bis 1000, bevorzugt 1 Volumenteile der Verdünnungslösung.

Überraschenderweise sind der erfindungsgemäßen parenteralen Lösungen schwer löslicher Arzneimittel stabil und können ohne Probleme angewendet werden.

<u>Beispiel 1</u>

0,32 kg Nafazatrom und 0,40 kg Natriummetabisulfit werden unter Rühren und Stickstoffbegasung in 60,00 kg Tetrahydrofurfurylalkoholpolyethylenglykolether und 30,00 kg Wasser für Injektionszwecke gelöst und mit Wasser für Injektionszwecke auf 100 Liter aufgefüllt. Nach Filtration werden unter Stickstoffbegasung jeweils 2,5 ml in Ampullen abgefüllt und hitzesterilisiert (Konzentrat).

5,5 kg Oxypolygelatine, 0,584 kg Natriumchlorid und 0,019 kg Ethylendiamintetraessigsäure (Dinatriumsalz) werden in 80 kg Wasser für Injektionszwecke gelöst und der Ansatz mit Wasser für Injektionszwecke auf 100 Liter aufgefüllt. Die Lösung wird filtriert, unter Stickstoffbegasung in Ampullen zu 7,5 ml abgefüllt und hitzesterilisiert (Verdünnungslösung).

Das Injektionssystem besteht aus einer Konzentratampulle und einer Ampulle mit Verdünnungslösung und ermöglicht die intravenöse Applikation von 8 mg Nafazatrom als klare, physiologisch verträgliche Lösung.

Nach Mischen von 2,5 ml Konzentrat und 7,5 ml Verdünnungslösung entsteht eine klare, übersättigte Injektionslösung.

Beispiel 2

0,05 kg Nimodipin werden in 30,0 kg Ethanol und 30,0 kg Polyethylenglykol 400 under Lichtschutz aufgelöst. Nach Auffüllen mit Wasser für Injektionszwecke auf 100 Liter wird filtriert und in Braunglasflaschen zu 50 ml unter aseptischen Bedingungen abgefüllt (Konzentratlösung).

Die Verdünnungslösung entspricht Beispiel 1.

Nach Mischen von 50 ml Konzentrat und 50 ml Verdünnungslösung entsteht eine applikationsfertige Infusionslösung.

Beispiel 3

0,06 kg 2-Methyl-4-(4-oxo-2-phenyl-4H-thiochromen-8-yl)-5-oxo-1,4,5,7-tetrahydrofuro[3,4-b]pyridin-3-carbonsäureethylester wird unter Lichtschutz in 24,00 kg Ethanol und 48,00 kg Polyethylenglykol 400 aufgelöst. Nach Auffüllen mit Wasser für Injektionszwecke auf 100 Liter wird filtriert und in Braunglasampullen zu 5 ml abgefüllt (Konzentratlösung).

4 kg Gelatine-polysuccinat mit einem mittleren Molekulargewicht von 30.000 und einem Succinylierungsgrad von 0,026, 0,3675 kg Natriumacetat-trihydrat, 0,1607 kg Natriumchlorid, 0,0403 kg Kaliumchlorid, 0,0133 kg Calciumchloriddihydrat, 0,0203 kg Magnesiumchloridhexahydrat, 0,02 kg Bernsteinsäure, 0,302 kg Natriumhydroxid und 0,517 kg konzentrierte Salzsäure werden in 90 kg Wasser für Injektionszwecke gelöst und mit Wasser für Injektionszwecke auf 100 Liter aufgefüllt. Nach Filtration wird die Lösung in Ampullen zu 5 ml abgefüllt und hitzesterilisiert (Verdünnungslösung).

Die applikationsfertige Injektionslösung mit 3 mg Wirkstoff in 10 ml Lösung ensteht durch Mischen einer Ampulle Konzentratlösung und einer Ampulle Verdünnungslösung.

Beispiel 4

0,04 kg Nitrendipin wird unter Lichtschutz in 20,00 kg Ethanol und 40,00 kg Polyethylenglykol 300 aufgelöst. Nach Auffüllen mit Wasser für Injektionszwecke auf 100 Liter wird filtriert und in Braunglasampullen zu 5 ml abgefüllt (Konzentratlösung).

3,5 kg Polygeline, 0,073 kg Calciumchlorid und 0,847 kg Natriumchlorid werden in 90 kg Wasser für Injektionszwecke gelöst und mit Wasser für Injektionszwecke auf 100 Liter aufgefüllt. Nach Filtration wird die Lösung in Ampullen zu 5 ml abgefüllt und hitzesterilisiert (Verdünnungslösung).

Die applikationsfertige Injektionslösung mit 2 mg Wirkstoff in 10 ml Lösung entsteht durch Mischen einer Ampulle Konzentratlösung und einer Ampulle Verdünnungslösung.

Anwendungsbeispiel:

Beispiel 5

Beim Verdünnen eines Konzentrates aus 8 mg Nafazatrom, 10 mg Natriummetabisulfit und 1,5 g Tetrahydrofurfurylalkoholpolyethylenglykolether mit Wasser tritt spontan Trübung ein. Wird zur Verdünnung hingegen eine 5,5 %ige Oxypolygelatinelösung verwendet, tritt eine Stabilisierung des übersättigten Zustandes ein und die Lösung bleibt klar (Tab. 1).

| | | | | | |
|---|---|---|---|---|---|
| **Zusammensetzung Konzentrat:** | | | | | |
| Nafazatrom | 5 mg | 6,5 mg | 8 mg | 9 mg | 10 mg |
| Natriummetabisulfit | 10 mg | 10 mg | 10 mg | 10 mg | 10 mg |
| Tetrahydrofurfurylalkohol-polyethylenglykolether | 1,50 g | 1,50 g | 1,50 g | 1,50 g | 1,50 g |
| **Verdünnung mit 8,7 g Wasser:** | | | | | |
| | klar | trüb | trüb | trüb | trüb |
| **Verdünnung mit 8,7 g 5,5 %iger Oxypolygelatinelösung:** | | | | | |
| | klar | klar | klar | klar | trüb |

Tab.1 Konzentrationsabhängigkeit der Klarheit von Nafazatrom-Injektionslösungen, die durch Verdünnen mit Wasser und einer 5,5 %igen Oxypolygelatinelösung hergestellt wurden.

**Ansprüche**

1. Parenterale Lösungen von schwer löslichen Arzneimitteln, enthaltend modifizierte Gelatine.

2. Parenterale Lösungen nach Anspruch 1, enthaltend Arzneimittel mit einer Löslichkeit in Wasser im Bereich von 1 µg bis 10 g pro Liter.

3. Parenterale Lösungen nach den Ansprüchen 1 und 2, bestehend aus einer das Arzneimittel enthaltenden Konzentratlösung und einer Verdünnungslösung.

4. Parenterale Lösungen nach den Ansprüchen 1 bis 3, dadurch gekennzeichnet, daß die Konzentratlösung 0,1 bis 30 % G/V modifizierte Gelatine enthält.

5. Parenterale Lösungen nach den Ansprüchen 1 bis 4, dadurch gekennzeichnet, daß die Verdünnungslösung 0,1 bis 30 % G/V modifizierte Gelatine enthält.

6. Parenterale Lösungen nach den Ansprüchen 1 bis 5, dadurch gekennzeichnet, daß modifizierte Gelatine mit einem mittleren Molekulargewicht im Bereich von 10.000 bis 50.000 verwendet wird.

7. Verfahren zur Herstellung von parenteralen Lösungen von schwer löslichen Arzneimitteln, dadurch gekenn zeichnet, daß man das Arzneimittel in einem Lösungsmittel löst und dann Wasser, modifizierte Gelatine und übliche Hilfsstoffe zusetzt.

8. Verfahren zur Herstellung von parenteralen Lösungen nach Anspruch 7, dadurch gekennzeichnet, daß man zur Herstellung der Konzentratlösung das Arzneimittel unter Rühren in einem organischen Lösungsmittel löst und dann Wasser, modifizierte Gelatine und übliche Hilfsstoffe zusetzt und zur Herstellung der Verdünnungslösung modifizierte Gelatine in Wasser löst und dann die Konzentratlösung und die Verdünnungslösung mischt.

9. Verfahren nach den Ansprüchen 7 und 8, dadurch gekennzeichnet, daß man 1 Volumenteil der Konzentratlösung mit 0,2 bis 1000 Volumenteile der Verdünnungslösung mischt.